# EUROPEAN PATENT APPLICATION

(11) **EP 2 298 393 A1**
(43) Date of publication of application: **23.03.2011**
(21) Application number: 09011814.2
(22) Date of filing: 16.09.2009
(51) Int. Cl.: A61M 5/315, A61M 5/00

(54) **Syringe and method for dispensing a liquid in a controllable manner**

(71) Applicant: Alomar, Hamad Mohammed, 11372 Riyadh (SA)
(72) Inventor: Alomar, Hamad Mohammed, 11372 Riyadh (SA)
(74) Representative: Appelt, Christian W.

(57) **Abstract**

The present invention relates to a syringe for dispensing a liquid in a controllable manner and a corresponding method.

The syringe according to the present invention comprises a body (10) having a first end (13), a second end (15), and a sidewall (11) extending in between said first and second ends; a plunger (14) comprising a plunger head (16), wherein said plunger head (16) is configured to be inserted into said body (10) via the second end (15) and wherein said plunger head (16) is configured to be moveable inside the body (10) to define a reservoir (18) of variable volume between said plunger head (16), said sidewall (11) and said first end (13) of said body (10); and a first element (26; 32) arranged on said sidewall (11) and a second element (28; 30) arranged on said plunger (14), wherein the first element (26; 32) is formed to provide a mating element for the second element (28; 30) and wherein the mating first and second elements (26, 28; 32, 30) are configured to engage with one another at a pre-defined position of said plunger (14) relative to said body (10). Said two mating elements are to dispense exact and pre-defined volumes of said liquid.

## Description

### Technical field

The present invention relates to a syringe for dispensing a liquid in a controllable manner and a corresponding method.

### Background art

Dispensing liquids by means of a syringe is a well known process. A typical syringe comprises a body in the form of a barrel holding said liquid to be dispensed, wherein a certain amount of said liquid inside the syringe is ejected by pressing on a plunger, which is partly inserted into said body at a proximal end of said syringe.

By pressing on the end of the plunger which protrudes from said syringe, e.g. by means of a user's finger, said plunger is moved inside said syringe to exert pressure on the liquid. Thereby, said liquid, in turn, is pressed through an outlet at the distal end of said syringe, wherein said outlet is typically fitted with a needle or the like.

The plunger may be moved up to a point at which substantially all the liquid has been dispensed, i.e. at which one end of said plunger is in contact with said distal end of the syringe from which the liquid is ejected.

Typically, the barrel of a known syringe is made from a transparent material. In addition, said barrel typically has visual marks arranged thereon, indicating different volume levels. Thus, by pushing the plunger towards the distal end of the syringe, the user may estimate the volume of liquid which has been dispensed.

Sometimes, however, it is important to know the exact volume of liquid which has been dispensed from said syringe. While, in some instances, it may suffice to roughly estimate said volume using the visual marks referred to above, it may not suffice in certain other instances, in which an exact knowledge of the dispensed volume is required. Sometimes, however, it is very difficult to certain users such as users with vision problems or older users to give the exact amount of said liquid. Sometimes, in case of medical emergency, it is difficult and time consuming to inject the exact required amount of said liquid to patients by the health care giver.

It is therefore an object of the present invention to provide an improved syringe for dispensing a liquid in a controllable manner as well as a corresponding method.

More specifically, it is an object of the present invention to provide a syringe and a method which enable the user to dispense an exact amount of liquid or even to dispense in subsequent steps several exact amounts of liquid.

### Disclosure of the invention

According to the present invention, the syringe comprises a body having a first end, a second end, and a side wall extending in between said first and second ends, a plunger comprising a plunger head, wherein said plunger head is configured to be inserted into said body via the second end and wherein said plunger head is configured to be moveable inside the body to define a reservoir of variable volume and configured to hold a liquid between said plunger head, said side wall and said first end of said body. Further, the syringe comprises a first element arranged on said side wall and a second element arranged on said plunger, wherein the first element is formed to provide a mating element for the second element and wherein the mating first and second elements are configured to engage with one another at a pre-defined position of said plunger relative to said body.

Thus, by providing for an engagement of said two mating elements at a pre-defined position of said plunger relative to said body, the user can press the plunger until reaching said pre-defined position. At this position, the user will feel an increased resistance due to the engagement of the two elements, which may preferably be a locking, latching, or catching engagement. Thereby, the user receives an immediate feedback as to the volume which has been dispensed so far or which is still left inside the reservoir.

In a particularly preferred embodiment of the present invention, the mating first and second elements are configured to engage with one another to form a releasable snap locking between the plunger and the body.

Preferably, said body has a cylindrical shape, i.e. comprises a cylindrical barrel of a circular cross-section.

The syringe may further comprise an outlet located at said first end of said body, wherein the outlet is preferably configured to allow a needle to be (releasably) attached thereto. The outlet may define the only egress from said reservoir through which egress said liquid may be dispensed.

Preferably, one of said mating elements comprises a recess or an indentation. Likewise, one of said mating elements may comprise a protrusion. In case said body has a circular cross-section, said recess may be a circumferential groove, whereas said protrusion may be a circumferential ring.

In a particularly preferred embodiment, said first element comprises a recess and said second element comprises a protrusion. In an equally preferred embodiment, said second element comprises a recess and said first element comprises a protrusion. In both cases, the protrusion is at least partly formed to inversely match the shape of at least a part of said recess or indentation. Thus, the first element is designed to engage with the second element. At least one of the elements, preferably the protrusion, may be formed from a resilient material to provide for a snap locking engagement.

Preferably, the syringe comprises several first elements arranged on said sidewall, wherein said second element is formed on said plunger head and wherein the second element is configured to engage with one of said mating first elements at a pre-defined position of said plunger relative to said body, wherein each first element defines such a position. The user may thus move the plunger from one pre-defined position to the next, in the course of which exact quantities of liquid are dispensed.

In an equally preferred embodiment, the syringe comprises several second elements arranged on said plunger, wherein the first element is arranged on said side wall and configured to engage with one of said mating second elements at a pre-defined position of said plunger relative to said body, wherein each second element defines such a position.

The first and second elements may be colour-coded to visually aid the user with dispensing the desired amount of liquid. For instance, in case of several first elements, each or at least adjacent ones of said first elements may have a different colour. Likewise, in case of several second elements, each or adjacent ones of those second elements may have a different colour.

The present invention also relates to a method of dispensing pre-defined quantities of liquid from a syringe. The method comprises the sequence of steps of (i) providing a syringe comprising a body and a plunger including a plunger head being moveable within said body to define a reservoir of variable volume within said body, (ii) moving said plunger to dispense liquid from said reservoir, and (iii) arranging said plunger relative to said body at a first pre-defined position by means of two mating elements engaging with one another, thereby defining a first volume of liquid left inside said reservoir.

In a preferred embodiment, the method further comprises the step of (iv) disengaging said two mating elements to eject further liquid from the syringe, wherein the step of disengaging said two mating elements preferably includes the step of releasing a snap locking between the plunger and the body. Preferably, the method further comprises the step of (v) moving said plunger to dispense (additional) liquid from said reservoir. The method may further comprise the step of (vi) arranging said plunger relative to said body at a second pre-defined position by means of two mating elements engaging with one another, thereby defining a second volume of liquid left inside said reservoir.

Preferably, the two mating elements engage with one another to form a releasable snap locking between the plunger and the body.

Preferably, the mating elements are colour-coded, as described above.

Further details and advantages of the present invention will become apparent from the following detailed description and the figures.

### Brief description of the figures

Fig. 1 shows a syringe according to the first embodiment.
Fig. 2 shows a perspective view of the syringe of Fig. 1.
Fig. 3 shows a close-up of the syringe of Fig. 1.
Fig. 4 shows a syringe according to the second embodiment.
Fig. 5 shows a perspective view of the syringe of Fig. 4.

### Detailed description of at least one way of carrying out the invention

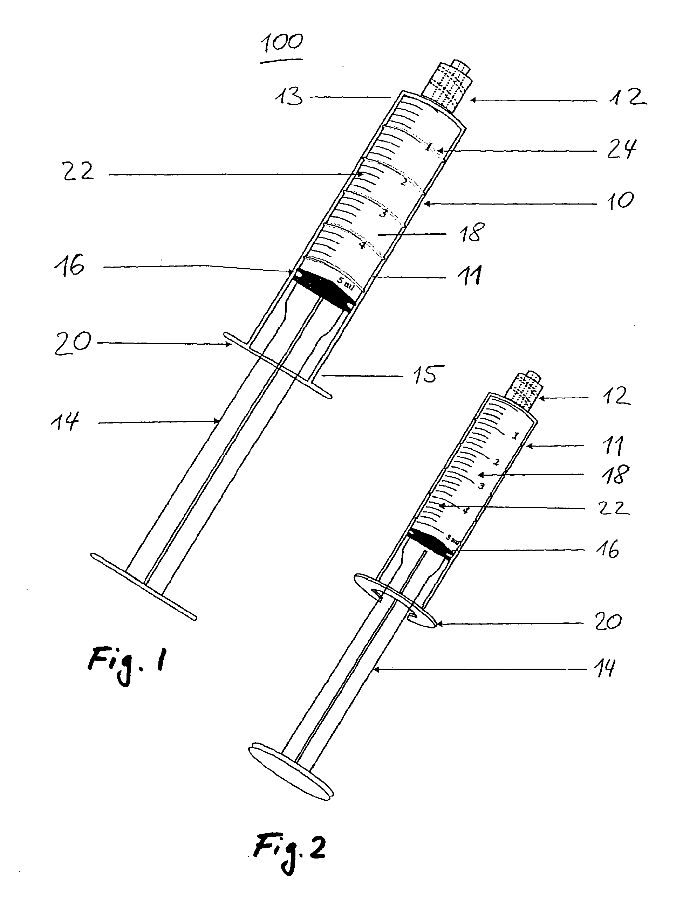

A syringe 100 according to the first embodiment of the present invention is shown in Fig. 1 to 3.

The syringe comprises a body 10 in the form of a cylinder the sidewall 11 of which having a circular cross-section. An outlet 12 is arranged at a distal end 13 of body 10. Said outlet 12 is fitted with threads to allow a needle to be releasably attached to said outlet 12 as well as distal end 13.

At the opposite, proximal end 15 of body 10, there is an opening through which a plunger 14 is inserted and received into the body. The plunger 14 has a plunger head 16 arranged at one end thereof. The head 16 has a round shape and substantially matches the inner diameter of the body 10.

The head 16 is configured to be moveable between the distal and proximal ends 13, 15 of said body 10 and to define a reservoir 18 between plunger head 16 and distal end 13 of the body 10. Said reservoir 18 holds the liquid to be dispensed from syringe 100.

At the proximal end of body 10, the sidewall 11 of said body 10 ends in a flange 20. As can be taken from Fig. 1, the user may press down the plunger 14 into body 10 using his thump by grasping body 10 near said flange 20 using the remaining fingers in order to dispense liquid from reservoir 18.

The sidewall 11 of body 10 is made of a transparent material. Visual marks 22, 24 in the form of calibration marks 22 and digits 24 are arranged on said sidewall 11 to allow the user to assess and gauge the volume of liquid which is left inside said reservoir 18.

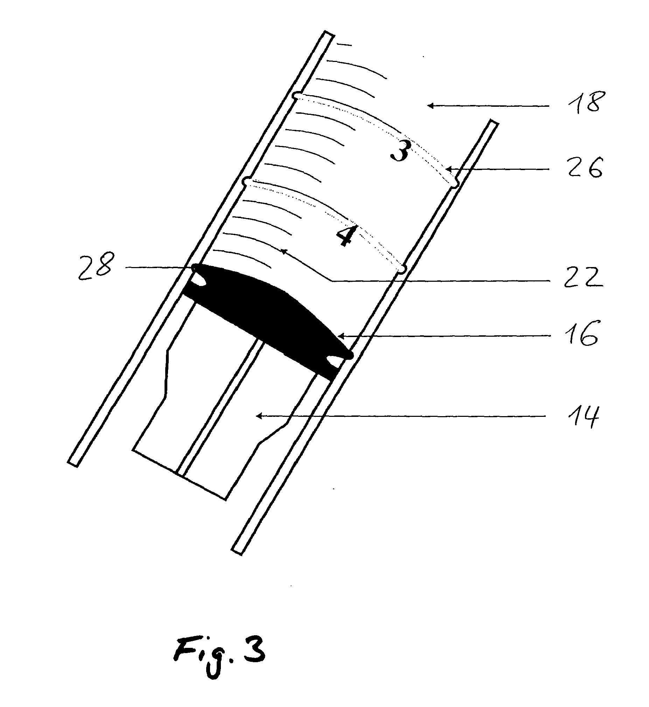

Turning to the close-up of the body 10 of the first embodiment shown in Fig. 3, the sidewall 11 is provided with several circumferential recesses 26 in the form of notches or grooves. The recesses 26 are arranged in parallel and are equally spaced in a longitudinal direction of body 10, i.e. between the distal and proximal ends 13, 15 of body 10. In the course of moving the plunger 14 and thus plunger head 16 from a first position, in which said plunger head 16 is aligned with a first recess 26, to a second position, in which said plunger head 16 is aligned with an adjacent, second recess 26, a pre-defined volume is dispensed. The present invention is, however, not limited to the recesses 26 being equally spaced. Further the recesses or at least adjacent ones thereof may have different colours to visually aid the user in dispensing a desired amount of liquid.

As shown in Fig. 3, the plunger head 16 comprises a ring-shaped, circumferential protrusion 28. Said protrusion 28 is formed of a resilient material, wherein said protrusion 28 is configured and shaped to contact the inside of sidewall 11 when positioned in between recesses 26 and to snap into engagement with one of the recesses 26 when positioned in alignment therewith. Every time ring-shaped protrusion 28 engages with a corresponding recess 26, the resistance against which the user pushes the plunger towards distal end 13 increases substantially. If the user stops pressing on the plunger 14 the very moment when the ring-shaped protrusion 28 of plunger head 16 forms a releasable snap-fit connection with a recess 26, the volume of dispensed liquid can be determined with high accuracy and exactitude.

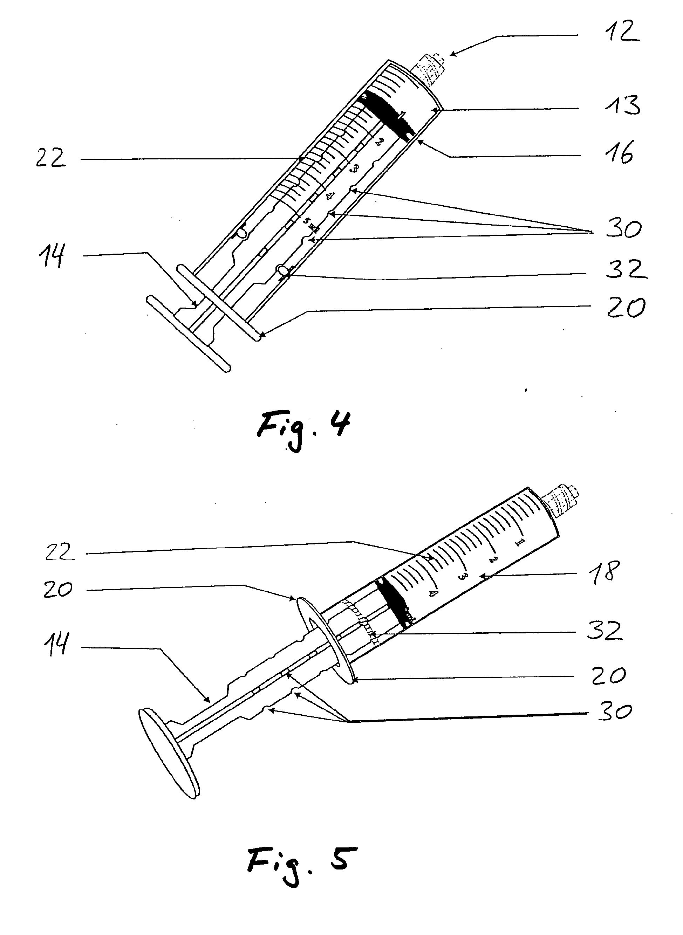

A second embodiment of a syringe according to the present invention is shown in Fig. 4 and Fig. 5.

The sidewalls 11 of the body 10 according to the second embodiment are substantially even on the inside thereof facing reservoir 18. It is thus possible for the plunger head 16 to move inside body 10, while being in contact with said sidewall 11 to retain the liquid inside reservoir 18.

Near the proximal end 15 of body 10 there is a circumferential protrusion 32 arranged on the inside of the sidewall 11 of body 10. Said protrusion 32 is of a ring-like shape. On the plunger 14 there are recesses 30 in the form of indentations, which are equally spaced in the longitudinal direction of said plunger 14 and may be colour-coded as described above in conjunction with the first embodiment. The shape of each of those recesses 30 on plunger 14 inversely matches the shape of the protrusion 32 on the inside of sidewall 11.

Said protrusion 32 is formed of a slightly resilient material, wherein said protrusion 28 is configured and shaped to be in contact with plunger 14 when positioned in between recesses 30 and to snap into engagement with one of the recesses 30 when positioned in alignment therewith. Every time ring-shaped protrusion 32 engages with a corresponding recess 30, the resistance against which the user pushes the plunger 14 towards distal end 13 increases substantially. If the user stops pressing on the plunger 14 the very moment when the ring-shaped protrusion 32 forms a releasable snap-fit connection with a recess 30, the volume of dispensed liquid can be determined with high accuracy and exactitude.

Either of the above described embodiments of the syringe may be used to effect a method of dispensing pre-defined quantities of liquid from said syringe comprising a body 10 and a plunger 14 including a plunger head 16 being moveable within said body to define a reservoir 18 of variable volume within said body 10. By exerting pressure on said plunger 14 said plunger head 16 is moved towards the distal end 13 of body 10 thereby dispensing liquid from said reservoir 18. As soon as said plunger 14 is arranged relative to said body 10 at a first position, where two mating elements engage with one another to form a snap-fit connection (i.e., protrusion 28 on the plunger head 16 and one of the recesses 26 on the inside sidewall 11 in the first embodiment or protrusion 32 on the inside of sidewall 11 and one of the recesses 30 on the plunger 14 in the second embodiment) a first volume of liquid is left inside said reservoir 18, which may be determined from the corresponding marks 22, 24 on the body 10.

Subsequently, the two mating and engaged elements can be disengaged again by exerting sufficient pressure on the plunger 14 to release the snap locking between the plunger 14 and the body 10 thereby allowing the user to push the plunger further towards the distal end 13 of syringe 100 and to dispense additional liquid from reservoir 18. As soon as plunger 14 is arranged relative to said body 10 at a second pre-defined position where once again two mating elements engage with one another to form a snap-fit connection, a second volume of liquid is left inside said reservoir 18, which may be determined from the corresponding mark 22, 24 on the body 10.

Thus, by moving the plunger from said first pre-defined position to said second pre-defined position, the user can determine with high accuracy the volume of liquid which has thus been dispensed.

The extent of the protection shall be determined by the claims, wherein the description and drawings shall be used to interpret the claims.

## Claims

1. Syringe, comprising
a body (10) having a first end (13), a second end (15), and a sidewall (11) extending in between said first and second ends;
a plunger (14) comprising a plunger head (16), wherein said plunger head (16) is configured to be inserted into said body (10) via the second end (15) and wherein said plunger head (16) is configured to be moveable inside the body (10) to define a reservoir (18) of variable volume between said plunger head (16), said sidewall (11) and said first end (13) of said body (10); and
a first element (26; 32) arranged on said sidewall (11) and a second element (28; 30) arranged on said plunger (14), wherein the first element (26; 32) is formed to provide a mating element for the second element (28; 30) and wherein the mating first and second elements (26, 28; 32, 30) are configured to engage with one another at a pre-defined position of said plunger (14) relative to said body (10).

2. Syringe according to claim 1, wherein the mating first and second elements (26, 28; 32, 30) are configured to engage with one another to form a releasable snap locking between the plunger (14) and the body (10).

3. Syringe according to claim 1 or 2, wherein said body (10) has a cylindrical shape.

4. Syringe according to one of the preceding claims, further comprising an outlet (12) located at said first end (13) of said body (10).

5. Syringe according to one of the preceding claims, wherein one of said mating elements (26; 30) comprises a recess.

6. Syringe according to one of the preceding claims, wherein the one of said mating elements (28; 32) comprises a protrusion.

7. Syringe according to one of the preceding claims, comprising several first elements (26) arranged on said sidewall (11), wherein said second element (28) is formed on said plunger head (14) and wherein the second element (28) is configured to engage with one of said mating first elements (26) at a pre-defined position of said plunger (14) relative to said body (10), wherein each of said first elements defines a different position of said plunger relative to said body.

8. Syringe according to one of claims 1 to 6, comprising several second elements (30) arranged on said plunger (14), wherein the first element (32) is configured to engage with one of said mating second elements (30) at a pre-defined position of said plunger (14) relative to said body (10), wherein each of said second elements (30) defines a different position of said plunger relative to said body.

9. Method of dispensing pre-defined quantities of liquid from a syringe, comprising the steps of
Providing a syringe comprising a body and a plunger including a plunger head being moveable within said body to define a reservoir of variable volume within said body;
Moving said plunger to dispense liquid from said reservoir; and
Arranging said plunger relative to said body at a first pre-defined position by means of two mating elements engaging with one another, thereby defining a first volume of liquid inside said reservoir.

10. Method according to claim 9, further comprising the step of
Disengaging said two mating elements.

11. Method according to claim 9 or 10, wherein the step of disengaging said two mating elements includes the step of releasing a snap locking between the plunger and the body.

12. Method according to one of claims 9 to 11, further comprising the step of Moving said plunger from said first pre-defined position to dispense liquid from said reservoir.

13. Method according to one of claims 9 to 12, further comprising the step of Arranging said plunger relative to said body at a second pre-defined position by means of two mating elements engaging with one another, thereby defining a second volume of liquid inside said reservoir.

14. Method according to one of claims 9 to 13, wherein the two mating elements engage with one another to form a releasable snap locking between the plunger and the body.

15. Method according to one of claims 9 to 14, wherein the mating elements are colour-coded to indicate the respective volume of liquid inside said reservoir, when two of those mating elements engage with one another.
